# EUROPEAN PATENT SPECIFICATION

(11) **EP 1 639 987 B1**
(45) Date of publication and mention of the grant of the patent: **12.08.2009**
(21) Application number: 04022876.9
(22) Date of filing: 24.09.2004
(51) Int. Cl.: A61K 6/00, A61K 6/08

(54) **Dental adhesive composition**
Dentalklebstoffzusammensetzung
Composition d'adhésif dentaire

(43) Date of publication of application: 29.03.2006
(73) Proprietor: 3M ESPE AG, 82229 Seefeld (DE)
(72) Inventor: Thalacker, Christoph, Dr., 82362 Weilheim (DE); Heumann, Andreas, 86916 Kaufering (DE)

(56) References cited:
- EP-A1- 1 498 098
- WO-A-03/057792
- WO-A1-03/011232

## Description

The present invention relates to a dental adhesive composition. More specifically, it relates to a self-etching dental material comprising a carboxylic acid functional polymer. It can be used for adhering a dental restoration material to a tooth structure.

The restoration of dental structures often involves the adhesive securing of a dental material to the hard tissue. Another use for adhesives in dentistry is bonding orthodontic appliances to teeth. As many dental materials like resin composites of the art due to their chemistry do not adhere to hard tissue like dentin or enamel, a pre-treatment is necessary. This includes etching of the enamel surface with inorganic or organic acids, followed by priming and subsequent application of a bonding agent. In many cases, between such steps, one or more rinsing and drying steps are used. As a result, dental restoration and the application of orthodontic appliances typically involve multi-step procedures.

To simplify conventional restorative and/or orthodontic procedures, for example, it is desirable to provide compositions that accomplish two or more of the steps of etching, priming and/or bonding. Many of the adhesive compositions known from the art combine two or more of these steps.

A further important prerequisite for a long-lasting adhesive bond between the dental structure and the restorative material or orthodontic appliance is the formation of a homogeneous adhesive film on the dental structure. Poor wetting of the tooth structure by the adhesive, or a too low viscosity of the adhesive can result in an inhomogeneous adhesive film or pooling of the adhesive. Thus, many adhesive compositions known from the state of the art try to overcome this problem of poor rheological properties and/or a low viscosity.

Many current dental adhesives contain different viscosity modifiers or rheology additives such as fumed silica or polymeric thickeners.

US 4,952,613 (Kuraray) describes dental compositions comprising an organic carboxylic acid, a metal chloride and water. The dental composition could further comprise a thixotropic agent selected from the group consisting of high molecular weight thickeners of polyvinylpyrrolidone and carboxymethylcellulose, and a highly dispersable silica.

EP 1 287 805 A1 (GC) describes a one-pack type dental composition comprised of a polymerizable composition containing among others 1-5 % by weight of a polymerizable monomer containing a phosphoric acid group, 10-40 % by weight of a polymerizable monomer containing a plurality of carboxyl groups in one molecule and among others 15 - 50 % by weight of water and a viscosity modifier having a mean particle size of 0.01-0.05 µm. Suitable viscosity modifiers are ultra fine inorganic fillers such as fumed silica, aluminum oxide and titanium dioxide or a glass powder.

US 6,506,816 B1 (3M) describes dental resin cement materials having unique handling properties that comprise a filler, a polymerizable resin and a polymeric handling modifier which is dispersed in the polymerizable resin.

There are some materials that can exhibit a change in the rheological behavior after mixing of two or more components:
US 4,533,701 (Tokuyama Soda) describes an adhesive coating material with high water resistance. The material comprises (1) a polymer including two carboxyl groups or one carboxylic anhydride group, and (2) an organic titanate. The carboxyl groups or the carboxylic anhydride group is bonded to adjacent carbon atoms of the polymer. Since the two carboxyl groups or one carboxylic anhydride group form a bridge of a high strength with the organic titanate compound, an especially high water resistance is given to this material. As known by a person skilled from the art the combination of these compounds can lead to gelation and to an increased viscosity of the material.
US 4,648,844 (Kuraray) describes two pack type dental lining compositions comprising (a) a solution of a polymer having at least 10 mole % of a carboxyl-containing vinyl monomer, and (b) a solution of a zirconium chelate compound.
US 6,583,197 B1 (Shofu) describes a dental adhesive composition comprising among others (a) a polymerizable unsaturated monomer containing 5 % by weight of a radical polymerizable monomer having an acid group, and (b) an acid-reactive filler. An acid-base reaction between these compounds leads to a gradual increase in the viscosity.
US 5,256,447 (3M) describes a method of adhering a restorative material to a substrate using an adhesive composition. The adhesive composition comprises an ethylenically unsaturated phosphorylated compound, a carboxylic acid functional polymer, and a curing agent. It is used to adhere an amalgam to hard tissue such as dentin or enamel. As a conventional adjuvant the composition can also contain viscosity modifiers. The disclosed composition does not contain any water.
US 5,922,786 (3M) discloses a multiple-part dental adhesive primer composition that gives high adhesion values without the need for a separate acid etching step. The composition provides a Part A comprising an acidic polymerizable compound and a polymerizable diluent wherein the pH of Part A is greater than about 2. It further provides a Part B comprising an acidic material wherein the pH of Part B is below about 2. The composition contains 0.5 to 90 % by weight of water in Part A or in Part B or in both parts.

WO 03/011232 A1 discloses a resin-modified glass ionomer cement comprising a polymer having a plurality of acidic repeating units but being substantially free of polymerizable vinyl groups, a polymer having a plurality of acidic repeating units and a plurality of of polymerizable vinyl groups, a fluoroaluminosilicate glass, a redox cure system that can initiate dark cure of the vinyl groups and water.

In WO 03/057792 A2 a composition containing a polymerizable reducing agent is discribed. This reducing agent includes a functional group of urea or thiourea.

An adhesive composition witih decreased polarity upon polymerization ins disclosed in EP 1 498 098 A1. This composition comprises one or a mixture of phosphoric acid esters bearing substituents with one ethylenically unsaturated moiety on the phosphorus atom, one or a mixture of phosphoric acid esters bearing substituents with two or more ethylenically unsaturated moieties on the phosphorus atom, initiators, unsaturated monomers, and optionally an unsaturated polymer.

The dental adhesives with rheology modifiers or thickeners described above suffer from several drawbacks: If particulate viscosity modifiers or rheology additives as fumed silica are used, upon standing of the composition, gravitational separation of the particles may occur. Moreover, they have to be dispersed in the liquid in an extra step rendering the production process more expensive. The other viscosity modifiers described above , e.g. polyvinylpyrrolidone and carboxymethylcellulose, often do not copolymerize with the other components of the formulation, which might lead to an impaired performance of the adhesive. Moreover, if the adhesive is prepared by mixing two or more compositions, as most of the known self-etching adhesives, the use of too viscous compositions can lead to dosing and mixing problems, and to the incorporation of air bubbles.

Therefore it is an object of the present invention to provide a dental composition for improved adhering of a restorative material and/or an orthodontic appliance to a substrate surface e.g. dental structure, such as dentin, enamel, bone, or other hard tissue that avoids one or more of the problems mentioned above.

A further object is to provide a self-etching dental adhesive with improved properties, especially a self-etching dental composition with desirable rheological properties and/or viscosity while avoiding particulate viscosity modifiers.

Thus, the aforementioned objects can be achieved by providing a dental composition as described in the text below.

This invention provides a dental composition comprising: (a) at least one carboxylic acid functional polymer, (b) at least one acid derivative with stronger acidity than the carboxylic acid functional polymer (a), substituted with at least one polymerizable ethylenically unsaturated group, (c) 0.1 to 10 weight % of water. The carboxylic acid functional polymer (a) and the acid derivative (b) are present at least in an amount effective that the dental composition exhibit shear thinning and a viscosity from 1.0 to 20 Pa.s, when measured with a plate/plate geometry and at a shear rate of 0.5 to 1 s ⁻¹. The methods of viscosity measurements are described below.

It has been found that a composition with a carboxylic acid functional polymer (a) which may be ethylenically unsaturated, an acid derivative (b) with stronger acidity than the carboxylic acid functional polymer, which may be ethylenically unsaturated and 0.1 to 10 weight % of water can be provided which shows an increased viscosity compared to the compositions know from the art and/or shear thinning behavior.

In a preferred embodiment, the invention relates to a dental composition, comprising: (a) 1 to 30 weight % of at least one carboxylic acid functional polymer, substituted with at least one polymerizable ethylenically unsaturated group, (b) 10 to 80 weight % of at least one acid derivative with stronger acidity than the carboxylic acid functional polymer (a), substituted with at least one polymerizable ethylenically unsaturated group, (c) 0.1 to 10 weight % of water, (d) 1 to 80 weight % of at least one unsaturated monomer and/or prepolymer, (e) 0.1 to 10 weight % of initiators, stabilizers, wherein the carboxylic acid functional polymer (a) and the acid derivative (b) are present at least in an amount effective that the dental composition exhibit shear thinning and a viscosity from 1.0 to 20 Pa·s, when measured with a plate/plate geometry and at a shear rate of 0.5 to 1 s⁻¹.

The described dental composition comprises 0.1 to 10 weight % of water. For higher water contents, the viscosity becomes too low and the desired viscosity and/or shear thinning behavior can not be obtained. Preferably, the dental composition of the invention comprises 0.1 to 6 weight % of water.

Preferably, the dental composition of the invention shows a shear thinning behavior. Thus, the viscosity of the dental composition at a shear rate of 0.5 to 1 s ⁻¹ is from 1.0 to 20 Pas and the viscosity at a shear rate of 1000 s ⁻¹ is less than 1.0 Pa s. Alternatively or additionally, the viscosity of the dental composition at a shear rate of 1000 s⁻¹ is less than 30% of the viscosity at a shear rate of 0.5 s⁻¹. A composition with shear thinning behavior like this embodiment of the present invention is advantageous as it exhibits better handling properties and it allows a uniform wetting of dental structures.

It is a further aspect of the invention that the composition in a preferred embodiment may not comprise any particulate viscosity modifiers such as fumed silica, aluminum oxide and titanium dioxide or a glass powder.

In a preferred material, the dental composition comprises a carboxylic acid functional polymer (a) according to the following formula (I):

B(X)ₘ(Y)ₙ (I)

wherein B represents an organic backbone, each X independently is a carboxylic group, each Y independently is a polymerizable group, m is a number having an average value of 2 or more, and n is a number having an average value of 0 or more.
Preferably the backbone B is an oligomeric or polymeric backbone of carbon-carbon bonds, optionally containing substituents which do not unduly interfere with the polymerization reaction such as oxygen, nitrogen or sulfur heteroatoms. Suitable Y groups include, but are not limited to, substituted and unsubstituted acrylates, methacrylates, alkenes and acrylamides. The weight average molecular weight of the compound according the formula (I) is at least about 250, preferably between about 500 and 500,000 and more preferably between about 1,000 and 100,000.

Preferably, the carboxylic acid functional polymer (a) is selected from the group consisting of homopolymers and copolymers of unsaturated mono-, di- or tricarboxylic acids and/or their anhydrides optionally substituted with at least one ethylenically unsaturated group. This includes, for example, homopolymers of poly(meth)acrylic acid, copolymers of (meth)acrylic and itaconic acid, (meth)acrylic and maleic acid, methyl vinyl ether and maleic anhydride or maleic acid, ethylene and maleic anhydride or maleic acid, and styrene and maleic anhydride or maleic acid, each polymer having at least one acidic group substituted with a polymerizable ethylenically unsaturated group.

Examples of carboxylic acid functional polymers, optionally substituted with polymerizable ethylenically unsaturated groups according to component (a) include, but are not limited to homopolymers and copolymers of unsaturated mono-, di-,or tricarboxylic acids commonly used to prepare glass ionomer cements. Representative polyalkenoic acids are described, for example, in U.S. Pat. Nos. 3,655,605; 4,016,124; 4,089,830; 4,143,018; 4,342,677; 4,360,605 and 4,376,835. Particularly preferred polyalkenoic acids also include homopolymers of polyacrylic acid, and copolymers of acrylic and itaconic acids, acrylic and maleic acids, methyl vinyl ether and maleic anhydride or maleic acid, ethylene and maleic anhydride or maleic acid, and styrene and maleic anhydride or maleic acid. Particularly preferred carboxylic acid functional polymers substituted with polymerizable ethylenically unsaturated groups are (meth)acrylate functionalized copolymers of acrylic acid, (meth)acrylic acid, maleic acid, and itaconic acid as described e.g. in EP 0 323 120 B1. Mixtures of such polymers can be used if desired.

The carboxylic acid functional polymer (a) is preferably present in an amount of 2 - 15 parts by weight, preferably 2 - 10 parts by weight, more preferably 3 - 8 parts by weight.

Preferably, the acid functionality of the acid derivative (b) with stronger acidity than the carboxylic acid functional polymer (a), substituted with one or more polymerizable ethylenically unsaturated groups is selected from oxyacids or thio-oxy acids of B, C, N, S, P having a pKa value of less than 4.75. If desired, a precursor to the acid such as an acid anhydride, or ester can be used in place of the acid itself, e.g., to generate the desired acid in situ. Suitable polymerizable ethylenically unsaturated groups include, but are not limited to, substituted and unsubstituted acrylates, methacrylates, alkenes and acrylamides.

Examples of acid derivatives with stronger acidity than the carboxylic acid functional polymer (a), substituted with one or more polymerizable ethylenically unsaturated groups according to component (b) include, but are not limited to 2-methacryloyloxyethyl phosphate, 2-methacryloyloxypropyl phosphate, 3-methacryloyloxypropyl phosphate, 2-methacryloyloxybutyl phosphate, 3-methacryloyloxybutyl phosphate, 4-methacryloyloxybutyl phosphate, 5-methacryloyloxy-3-oxa-pentyl phosphate, bis(2-methacryloyloxyethyl) phosphate, bis(2-methacryloyloxypropyl) phosphate, bis(3-methacryloyloxypropyl) phosphate, bis(2-methacryloyloxybutyl) phosphate, bis(3-methacryloyloxybutyl) phosphate, bis(4-methacryloyloxybutyl) phosphate, bis(5-methacryloyloxy-3-oxa-pentyl) phosphate, glycerol-1,3-dimethacrylate-2-phosphate, glycerol-1,2-dimethacrylate-3-phosphate, bis(glycerol-1,3-dimethacrylate) phosphate, bis(glycerol-1,2-dimethacrylate) phosphate, (glycerol-1,2-dimethacrylate)(glycerol-1,3-dimethacrylate) phosphate, (trimethylolpropane dimethacrylate) phosphate, bis(trimethylolpropane dimethacrylate) phosphate, (trimethylolethane dimethacrylate) phosphate, bis(trimethylolethane dimethacrylate) phosphate, pentaerythritol trimethacrylate phosphate, maleic acid monoesters such as mono(methacryloyloxyethyl)maleate, sulfoethyl methacrylate, sulfopropyl methacrylate, 2-acrylamido-2-methylpropanesulfonic acid, and mixtures thereof.

The acid derivative (b) is present in an amount of 10 - 80 parts by weight of the total composition. Preferably, (b) is present in an amount of 20 - 80, more preferably 20 - 60 parts by weight.

The amount of (a) and (b) in the total composition is 10 - 90 parts by weight, preferably 15 - 80 parts by weight, more preferably 20 - 70 parts by weight.

The dental composition of the invention optionally comprises 1 to 80 weight % of at least one unsaturated monomer and/or prepolymer.
Examples for these unsaturated monomers are ethylene glycol di(meth)acrylate, diethylene glycol di(meth)acrylate, triethylene glycol di(meth)acrylate, tetraethylene glycol di(meth)acrylate, polyethylene glycol di(meth)acrylate, 1,3-butylene glycol di(meth)acrylate, 1,6-hexane diol di(meth)acrylate, neopentyl glycol di(meth)acrylate, tripropylene glycol di(meth)acrylate, polypropylene glycol di(meth)acrylate, glycerol di(meth)acrylate, bisphenol A di(meth)acrylate, bisphenol A glycidyl di(meth)acrylate, bisphenol A propyl di(meth)acrylate, bisphenol A isopropyl di(meth)acrylate, ethylene oxide modified bisphenol A di(meth)acrylate, ethylene oxide modified bisphenol A glycidyl di(meth)acrylate, 2,2-bis(4- methacryloxypropoxyphenyl) propane, 7,7,9-trimethyl-4,13-dioxy-3,14-dioxa-5,12-diazahexadecane-1,16-diol di(meth)acrylate, neopentyl glycol hydroxypivalic acid ester di(meth)acrylate, caprolactone modified hydroxypivalic acid neopentyl glycol ester di(meth)acrylate, trimethylol ethane di(meth)acrylate, trimethylol propane di(meth)acrylate, trimethylol methane tri(meth)acrylate, trimethylol ethane tri(meth)acrylate, trimethylol propane tri(meth)acrylate, pentaerythritol tri(meth)acrylate, dipentaerythritol tri(meth)acrylate, pentaerythritol tetra(meth)acrylate, dipentaerythritol tetra(meth)acrylate, dipentaerythritol hexa(meth)acrylate, the reaction product of 3-chloro-2-hydroxypropyl (meth)acrylate and methylcyclohexane diisocyanate, the reaction product of 2-hydroxypropyl (meth)acrylate and methylcyclohexane diisocyanate, the reaction product of 2-hydroxypropyl (meth)acrylate and methylene bis (4-cyclohexylisocyanate), the reaction product of 2-hydroxypropyl(meth)acrylate and trimethylhexamethylene diisocyanate, the reaction product of 2-hydroxyethyl (meth)acrylate and isophorone diisocyanate, and the reaction product of 3-chloro-2-hydroxypropyl (meth)acrylate and isophorone diisocyanate, methyl (meth)acrylate, ethyl (meth)acrylate, propyl methacrylate, isopropyl methacrylate, n-butyl (meth)acrylate, iso-butyl (meth)acrylate, 2-ethylhexyl (meth)acrylate, lauryl (meth)acrylate, tridecyl (meth)acrylate, stearyl (meth)acrylate, cyclohexyl (meth)acrylate, benzyl (meth)acrylate, 2-hydroxyethyl (meth)acrylate, 2-hydroxypropyl (meth)acrylate, glycidyl (meth)acrylate, tetrahydrofurfuryl (meth)acrylate, allyl (meth)acrylate, 2-ethoxyethyl (meth)acrylate, methoxydiethylene glycol (meth)acrylate, methoxytetraethylene glycol (meth)acrylate, methoxypolyethylene glycol (meth)acrylate, phenoxydiethyleneglycol (meth)acrylate, phenoxyhexaethyleneglycol (meth)acrylate, glycerol (meth)acrylate, tetrahydrofurfuryl (meth)acrylate, dicyclopentenyl (meth)acrylate, isobornyl (meth)acrylate, phenyl (meth)acrylate, pentaerythritol mono(meth)acrylate, dipentaerythritol mono(meth)acrylate, and mixtures thereof.

Examples of prepolymers, especially unsaturated urethane prepolymers and their preparation, are described in WO 01/ 44338 A1. The prepolymers preferably do not contain hydroxy, acidic or ionic groups.

Another particularly preferred unsaturated prepolymer is an urethane functionalized polymer. This can be obtained for example by reaction of (A) 15 to 85 wt.-% of one ore more α, ω-terminated poly(meth)acrylate diols, (B) 0 to 30 wt.-% of one or more radically curable, polyhydroxy-functional compounds, (C) 14 to 60 wt.-% of one ore more polyisocyanates, (D) 1 to 40 wt.% of a monofunctional compound, reactive vis-à-vis isocyanate groups, which contain one or more radically curable groupings. The prepolymers obtained have an average molecular weight (Mw) according to GPC measurements against polystyrene standards in the range between 400 and 200.000 g/mol, preferably between 500 and 100.000 g/mol and more preferably between 600 and 20.000 g/mol. An example is the step growth polymerization product of Tego® Diol BD-1000, 2,4,4-trimethylhexyl-1,6-diisocyanate and 2-hydroxyethylmethacrylate. This is commercially available from Tego Chemie Service GmbH, Germany.

The unsaturated monomers and/or prepolymers are present in an amount of 1 - 80 parts by weight, preferably 10 - 50 parts by weight, more preferably 20 - 45 parts by weight,

Moreover, it has been found that adding urethane or amide group containing monomers, prepolymers, and/or polymers to the dental composition of the invention leads to preferred compositions with improved properties like miscibility or wetting of a dental structure.

Initiators according to the invention are curing agents like photoinitiators or photoinitiator systems for free radical polymerization known to the person skilled in the art.

Suitable photoinitiators for polymerizing free radically photopolymerizable compositions include the class of phosphine oxides. Preferred phosphine oxide free radical initiators are acyl and bisacyl phosphine oxides such as those described in U.S. Pat. Nos. 4,298,738; 4,324,744; 4,385,109; 4,710,523; and 4,737,593; 6,251,963; and EP Application No. 0 173 567 A2.
Commercially available phosphine oxide photoinitiators capable of free-radical initiation when irradiated at wavelength ranges of greater than 380 nm to 450 nm include bis(2,4,6-trimethylbenzoyl)phenyl phosphine oxide (IRGACURE 819, Ciba Specialty Chemicals), bis(2,6-dimethoxybenzoyl)-(2,4,4-trimethylpentyl) phosphine oxide (CGI 403, Ciba Specialty Chemicals), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700, Ciba Specialty Chemicals), a 1:1 mixture, by weight, of bis(2,4,6-trimethylbenzoyl)phenyl phosphine oxide and 2-hydroxy-2-methyl-1-phenylpropane-1-one (DAROCUR 4265, Ciba Specialty Chemicals), and ethyl 2,4,6-trimethylbenzylphenyl phosphinate (LUCIRIN LR8893X, BASF).
Other typical examples are combinations of photoinitiators or photoinitiator systems for free radical polymerization include combinations of a sensitizing agent with a reducing agent.

As the sensitizing agent, those which can polymerize the polymerizable monomer by the action of a visible light having a wavelength of from 390 nm to 830 nm are preferred. Examples thereof include camphorquinone, benzil, furil, 3,3,6,6-tetramethylcyclohexanedione, diacetyl, benzyl dimethyl ketal, benzyl diethyl ketal, benzyl di(2-methoxyethyl) ketal, 4,4,'-dimethylbenzyl dimethyl ketal, anthraquinone, phenanthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropyl thioxanthone, 2-nitrothioxanthone, 2-methyl thioxanthone, 2,4-dimethyl thioxanthone, 2,4-diethyl thioxanthone, 2,4-diisopropyl thioxanthone, 2- chloro-7-trifluoromethyl thioxanthone, thioxanthone-10,10-dioxide, thioxanthone-10-oxide, benzoin methyl ether, benzoin ethyl ether, isopropyl ether, benzoin isobutyl ether, benzophenone, bis(4-dimethylaminophenyl)ketone, 4,4,'-bisdiethylaminobenzophenone, acyl phosphine oxides such as (2,4,6-trimethylbenzoyl)diphenylphosphine oxide, and azide-containing compounds. These compounds may be used singly or in admixture.
As the reducing agent, tertiary amines and the like are generally used. Suitable examples of the tertiary amines include N,N-dimethyl-p- toluidine, N,N-dimethylaminoethyl methacrylate, triethanolamine, methyl 4-dimethylaminobenzoate, ethyl 4-dimethylaminobenzoate, and isoamyl 4-dimethylaminobenzoate. As other reducing agents, sodium sulfinate derivatives and organometallic compounds can also be used. These compounds may be used singly or in admixture. If desired, a combination of an initiator system including a sensitizer and a reducing agent with a phosphine oxide initiator can be used.
Further examples of suitable initiator systems can be found in the literature, e.g. J.-P. Fouassier, Photoinitiation, Photopolymerization, and Photocuring, Hanser Publishers, Munich, Vienna, New York, 1995, and J.-P. Fouassier, J.F. Rabek (eds.), Radiation Curing in Polymer Science and Technology, Vol. II, Elsevier Applied Science, London, New York, 1993.
Moreover, ternary photopolymerization initiating systems consisting of a sensitizer, an electron donor and an onium salt as described in US 6,187,833; US 6,025,406; US 6,043,295; US 5,998,495; US 6,084,004 and US patent application Serial No. 10/050218 can be used.

The initiators are present in an amount of 0.1 - 10 parts by weight, preferably 0.2 - 10 parts by weight, more preferably 0.3 - 5 parts by weight, according to the total composition.

Examples of stabilizers according to the invention are butylated hydroxytoluene (BHT), hydroquinone, hydroquinone monomethyl ether (MEHQ), 3,5-di-tert-butyl-4-hydroxyanisole (2,6-di-tert-butyl-4-ethoxyphenol), 2,6-di-tert-butyl-4-(dimethylamino)methylphenol or 2,5-di-tert-butyl hydroquinone, 2-(2'-hydroxy-5'-methylphenyl)-2H-benzotriazole, 2-(2'-hydroxy-5'-t-octylphenyl)-2H-benzotriazole, 2-hydroxy-4-methoxybenzophenone (UV-9), 2-(2'-hydroxy-4',6'-di-tert-pentylphenyl)-2H-benzotriazole, 2-hydroxy-4-n-octoxybenzophenone, and 2-(2'-hydroxy-5'-methacryloxyethylphenyl)-2H-benzotriazole. Such adjuvants may optionally comprise reactive functionality so that they will be copolymerized with the resin.

Preferably, the stabilizers are present in an amount of 0 - 5 parts by weight, more preferably 0.001 - 2 parts by weight, most preferably 0.01 - 1 parts by weight.

The dental composition of the invention may further contain commonly used auxiliaries like, for example, surfactants, solvents, fluoride releasing agents, non reactive inorganic fillers and photobleachable colorants.

Dental compositions of the present invention optionally contain surfactants including nonionic surfactants, anionic surfactants, cationic surfactants, amphoteric surfactants, and combinations thereof. Surfactants useful in compositions and methods of the present invention include non-polymerizable and polymerizable surfactants.
The nonionic surfactants are usually condensation products of an organic aliphatic or alkylaromatic hydrophobic compound and an alkylene oxide, such as ethylene oxide, which is hydrophilic. Almost any hydrophobic compound having a carboxy, hydroxy, amido, or amino group with a free hydrogen present can be condensed with ethylene oxide to form a nonionic surfactant.
Particularly suitable nonreactive nonionic surfactants include but are not limited to those selected from the group consisting of the condensation products of a higher aliphatic alcohol, such as a fatty alcohol, containing about 8 to about 20 carbon atoms, in a straight or branched chain configuration, condensed with about 3 to about 100 moles, preferably about 5 to about 50 moles, most preferably about 5 to about 40 moles of ethylene oxide. Examples of such nonionic ethoxylated fatty alcohol surfactants are the Tergitol 15-S series from Dow and Brij surfactants from Uniqema. Brij 35 Surfactant is Polyoxyethylene(23) lauryl ether.
Other suitable nonreactive nonionic surfactants include but are not limited to those selected from the group consisting of the polyethylene oxide condensates of one mole of alkyl phenol containing from about 6 to 12 carbon atoms in a straight or branched chain configuration, with about 3 to about 100 moles, preferably about 5 to about 50 moles, most preferably about 5 to about 40 moles of ethylene oxide. Examples of such nonreactive nonionic surfactants are the Tergitol NP and Triton X series from Dow and Igepal CO and CA series from Rhodia. Tergitol NP and Igepal CO surfactants include nonylphenoxy poly(ethyleneoxy) ethanols. Triton X and Igepal CA surfactants include octylphenoxy poly(ethyleneoxy) ethanols.
Another group of usable nonreactive nonionic surfactants include but are not limited to those selected from the group consisting of block copolymers of ethylene oxide and propylene oxide or butylene oxide. Examples of such nonionic block copolymer surfactants are the Pluronic and Tetronic series of surfactants from BASF, and the Synperonic series of surfactants from Uniqema.
Still other useful nonreactive nonionic surfactants include but are not limited to those selected from the group consisting of sorbitan fatty acid esters, polyoxyethylene sorbitan fatty acid esters and polyoxyethylene stearates. Examples of such fatty acid ester nonionic surfactants are the Span, Tween, and Myrj surfactants from Uniqema. Span surfactants include C₁₂ - C₁₈ sorbitan monoesters. Tween surfactants include poly(ethylene oxide) C₁₂ - C₁₈ sorbitan monoesters. Myrj surfactants include poly(ethylene oxide) stearates.

Useful polymerizable non-ionic surfactants typically have polymerizable groups such as hydroxyethyl methacrylate, hydroxypropyl methacrylate, glycerol dimethacrylate, pentaerythritol triacrylate and the like that are attached (e.g., via a urethane linkage) to a hydrocarbon group (e.g., hexyl, cyclohexyl, pentyl, or octyl) to a polyethylenegycol chain. The methacrylate or acrylate moiety (hereafter written as (meth)acrylate) together with the hydrocarbon group moiety form a non polar hydrophobic end, while the polyethylene glycol chain forms a polar hydrophilic end. By varying the number of (meth)acrylate groups and the length or number of the polyethylene glycol or chains, surfactants with a wide variety of properties may be produced. In addition to the (meth)acrylate groups, other non polar groups such as fatty acids may also be incorporated to provide an even more hydrophobic end as desired. The polyethylene glycol chain may further be broken into several short chains if desired.
Useful polymerizable nonionic surfactants include, for example, the polymerizable nonionic surfactants available under the Blemmer series from Nippon Oil and Fat, and the Noigen series from Dai-Ichi Kogyo Seiyaku.
Useful anionic surfactants include, for example, aliphatic metal carboxylates such as sodium laurate, sodium stearate and sodium oleate; sulfated aliphatic metal carboxylates such as sodium dioctylsulfosuccinic acid; metal salts of higher alcohol sulfates such as sodium dodecylsulfate, sodium lauryl sulfate, sodium cetyl sulfate, sodium stearyl sulfate and sodium oleyl sulfate; metal salts of higher alkylether sulfates such as sodium lauryl ether sulfate obtained by sulfating an ethylene oxide adduct with a lauryl alcohol; metal salts of alkylbenzenesulfonic acids such as sodium dodecylbenzenesulfonate; metal salts of alpha-olefinsulfonic acids synthesized by reacting sulfuric acid with an alpha-olefin; Igepon T obtained by reacting N-methyl taurine and oleic acid chloride; sulfosuccinic acid diesters typified by Aerosol OT; phosphoric acid ester salts of adducts of ethylene oxide with higher alcohols; and dithiophosphoric acid ester salts.
Useful cationic surfactants include, but are not limited to adducts of ethylene oxide with higher alkylamines such as stearylamine; amines prepared from lower amines; alkyltrimethyl ammonium salts such as lauryl trimethylammonium chloride; quaternary ammonium salts prepared from higher alkylamines typified by alkyldimethylbenzyl ammonium salts such as lauryl dimethylbenzyl ammonium chloride; Sapamine type quaternary ammonium salts; and quaternary ammonium salts prepared from lower amines typified by pyridinium salts such as Zelan AP and Velan PF.
Examples for polymerizable cationic surfactants include, but are not limited to methacryloyloxyalkyl ammonium salts, like for example methacryloyloxyethyl trimethylammonium chloride.
Examples for amphoteric surfactants are metal salts of higher alkylaminopropionic acids such as sodium laurylaminopropionate and sodium stearylaminopropionate; and betaines such as lauryl dimethylbetaine, stearyl dimethylbetaine and lauryl dihydroxyethylbetaine.
The surfactants may be present in an amount of 0.1 - 20 parts by weight, preferably 0.2 - 10 parts by weight, more preferably 0.3 - 5 parts by weight.

A preferred embodiment of the present invention includes a solvent. Examples of solvents include, but are not limited to linear, branched or cyclic, saturated or unsaturated alcohols with 2 to 10 C atoms, ketones, esters or mixtures of two or more of said type of solvents.
Especially preferred alcoholic solvents are methanol, ethanol, iso-propanol and n-propanol.
Other suitable organic solvents are THF, acetone, methylethyl ketone, cyclohexanol, toluene, alkanes and acetic acid alkyl esters, in particular acetic acid ethyl ester.
Generally, it is possible to use the above-mentioned solvents alone or as a mixture of two or more of any of these solvents, if the solvent mixtures do not impair the adhesive properties, the viscosity and / or the rheological properties to such an extent that the desired result cannot be obtained.

The solvent is present in an amount of 0 - 20 parts by weight, preferably 1 - 15 parts by weight, more preferably 3 - 10 parts by weight,

The dental composition may further comprise a fluoride release agent. Examples of a fluoride release agent are naturally occuring or synthetic fluoride minerals such as sodium fluoride, fluoride glass such as fluoroaluminosilicate glass, simple and complex inorganic fluoride salts such as potassium zinc fluoride and potassium hexafluorotitanate, simple and complex organic fluoride salts such as tetra ethyl ammonium tetra fluoroborate or combinations thereof. Optionally these fluoride sources can be treated with surface treatment agents.
Optionally, the fluoride release agent is present in an amount of 0 - 20 parts by weight, preferably 0.2 - 10 parts by weight, more preferably 0.3 - 5 parts by weight.

Further, a non reactive inorganic filler may be present. Examples of a nonreactive inorganic filler are naturally-occurring or synthetic materials such as quartz, nitrides (e.g., silicon nitride), glasses derived from, for example Ce, Sb, Sn, Zr, Sr, Ba or Al, colloidal silica, feldspar, borosilicate glass, kaolin, talc, titania, and zinc glass, zirconia-silica fillers; low Mohs hardness fillers such as those described in U.S. Pat. No. 4,695,251; and submicron silica particles (e.g., pyrogenic silicas such as the "Aerosil" Series "OX 50", "130", "150" and "200" silicas sold by Degussa, and "Cab-O-Sil M5" silica sold by Cabot Corp.). Preferred filler particles are quartz, submicron silica, and non-vitreous microparticles of the type described in US 4,503,169. Mixtures of these fillers are also contemplated, as well as combination fillers made from organic and inorganic materials.
Optionally, the surface of the filler particles may be treated with a surface treatment, such as a silane coupling agent, in order to enhance the bond between the filler and the polymerizable resin. The coupling agent may be functionalized with reactive curing groups, such as acrylates and/or methacrylates.
The non reactive inorganic filler can be present in an amount of 0 - 70 parts by weight, preferably 5 - 60 parts by weight, more preferably 10 - 50 parts by weight.

In certain instances, the fluoride source of the fluoride release agent and the filler can be the same.

Optionally a photobleachable colorant like for example Rose Bengal, Methylene Violet, Methylene Blue, Fluorescein, Eosin Yellow, Eosin Y, Ethyl Eosin, Eosin bluish, Eosin B, Erythrosin B, Erythrosin Yellowish Blend, Toluidine Blue, 4',5'-Dibromofluorescein and blends thereof may be present. Further examples of photobleachable colorants can be found in US 6,444,725. The color of the compositions of the present invention may be additionally imparted by a sensitizing compound.
The amount of the photobleachable colorant is 0 - 5 parts by weight, preferably 0.05 - 3 parts by weight, more preferably 0.1 - 2 parts by weight.

The optional components mentioned above might be present in the composition alone or in combination with the other optional components.

In another preferred embodiment, the composition of the present invention is provided as a kit of at least two parts, wherein one part A comprises
(a) at least one carboxylic acid functional polymer,
(aa) 0 to 10 weight % of water,
   and wherein another part B comprises
(b) at least one acid derivative with stronger acidity than the carboxylic acid functional polymer (a), substituted with at least one polymerizable ethylenically unsaturated group,
(bb) less than 0.5 weight % of water,
wherein the carboxylic acid functional polymer (a) and the acid derivative (b) are present at least in an amount effective that the mixture of the parts exhibit shear thinning and/or a viscosity, that is higher than that of each of the separate parts, when measured with a plate/plate geometry and at a shear rate of 0.5 to 1 s⁻¹.

Preferably the kit is provided with two parts, part A and part B. These parts can be provided in a ratio of A:B from 5:1 to 1:5, preferably in a ratio of A:B of 1:4.

It is therefore advantageous to mix two or more low viscous compositions in order to obtain a more viscous composition, which ideally exhibits shear thinning. In this case, ease of dosing, mixing, and application lead to a more robust, user friendly system.

The dental compositions of the invention can be used as dental adhesives, especially for the adhesive securing of dental filling materials, as self-adhesive dental pit and fissure sealants, as self-adhesive dental desensitizers, and as self-adhesive dental restorative materials

The dental compositions may be used for adhering of dental fillings based on methacrylates like materials described e.g. in WO 01/30305 A1, WO 01/30306 A1, WO 01/30307 A1.
Surprisingly it has been found that the dental composition of the present invention can be used for the adhesive securing of dental filling materials based on cationically polymerizable resins to a tooth or bone. Especially for cationically polymerizable resins like the epoxy resins and the silicon containing and polysiloxane epoxides an adhesive system was needed.
Suitable dental filling materials based on cationically polymerizable resins are described e.g. in US 6,254,828, US 6,084,004, WO 01/51540 A1, WO 02/66535 A1, WO 98/47046 A1, WO 98/47047 A1.

The dental compositions of the present invention provide at least comparable adhesion values to state of the art self-adhesive dental materials. In addition they exhibit an optimized rheological behavior (e.g. shear thinning) and/or an increased viscosity, and provide therefore better handling and, if applied for the adhesive securing of dental filling materials, a more homogeneous adhesive film without pooling or dewetting effects. Moreover, these effects are provided without the need for adding a particulate viscosity modifier (e.g. fumed silica). Thus, on the one hand, costly production steps like dispersing these particulate viscosity modifiers can be avoided, on the other hand, the danger of gravitational separation upon standing of the product can be eliminated. If the dental compositions of the present invention are provided as a kit of at least two parts, the composition of the desired rheological behavior and/or viscosity can be obtained by mixing two or more parts of lower viscosity thus providing clear advantages in terms of dosing, mixing, and application.

The inventive compositions are usually applied to the tooth surface in an amount sufficient to etch and prime the dental tissue. In this respect the following steps are applied:
a) applying the composition to the surface of a tooth (enamel and / or dentin), preferably using a brush or a sponge,
b) optionally dispersing the composition to a thin film, preferably using a stream of air,
c) light or redox initiated curing of the composition,
d) optionally applying a dental filling composition.
Optionally, one or more of the steps mentioned above can be repeated.

The composition can generally be provided in any type of package, e.g. tubes, or flasks. For the application of small amounts of liquids, however, the prior art discloses a number of alternatives which facilitate the application.
For dental application, multi chamber packaging devices as described in EP 0 895 943 B1, WO-02/38468 A1, WO 01/58869 A1 are preferred.

If the dental composition is provided in at least two parts, the partitioning of the components into the parts should prevent undesired reactions of the composition. Components comprising OH groups should preferably stored in one part, the acid derivatives (b) with stronger acidity than the carboxylic acid functional polymer in the other part.

The composition of the present invention usually is prepared by mixing the components, e.g. by stirring or shaking.

The enamel adhesion of the inventive dental composition is usually in the range of 2 - 40 MPa, preferably in the range of 5 - 35 MPa, more preferably in the range of 10 - 30 MPa, measured as described below.

The dentin adhesion of the inventive dental composition is usually in the range of 2 - 40 MPa, preferably in the range of 5 - 35 MPa, more preferably in the range of 10 - 30 MPa, measured as described below.
The viscosity of the inventive dental composition at a shear rate of 0.5 s⁻¹ is usually in the range of 1 - 20 Pa·s, preferably in the range of 1 - 15 Pa·s, measured as described below. If the composition is provided as a kit of at least two parts, the viscosity of the mixture of the parts is preferably equal or higher than the viscosities of the parts alone.
The viscosity vs. shear rate behavior of the inventive dental composition preferably exhibits shear thinning, i.e. decreasing viscosity with increasing shear rate.

### Examples

The invention is further described by the following examples. The examples are for illustrative purpose only and the particular materials and amounts thereof recited in these examples, as well as other conditions and details, should not be construed to unduly limit this invention. Unless otherwise indicated, all parts and percentages are on a weight basis, all water is deionized water, and all molecular weights are weight average molecular weight.

### Abbreviations:

- PM2: mixture of reaction products of 2-hydroxyethyl methacrylate (HEMA) with phosphorus pentoxide, commercially available from Nippon Kayaku as Kayamer PM2
- PGDMA: mixture of reaction products of glycerol di(methacrylate) (GDMA) with phosphorus pentoxide
- DBSA: dodecylbenzene sulfonic acid
- SEMA: 2-sulfoethyl methacrylate
- MEMA: mono(methacryloyloxyethyl)maleate
- AA: glacial acetic acid
- HEMA: hydroxyethyl methacrylate
- TEGDMA: triethylene glycol dimethacrylate
- TMHDI: 2,4,4-trimethylhexyl-1,6-diisocyanate
- PUMA: step growth polymerization product of Tego® Diol BD-1000, TMHDI, and HEMA (preparative example 2 of WO 01/44338 A1)
- BisEMA: ethoxylated bisphenol A dimethacrylate (with a mean of 4 ethylene oxide units per molecule)
- BPDMA: bisphenol A dipropylmethacrylate
- VBP: the precipitated dry polymer of example 11 of EP 0 323 120 B1
- CPQ: camphorquinone
- EDMAB: ethyl 4-dimethylaminobenzoate
- DPIPF6: diphenyliodonium hexafluorophosphate
- Brij 35: polyoxyethylene(23) lauryl ether
- MEHQ: hydroquinone monomethyl ether
- BHT: butylated hydroxytoluene

### Test Methods

### Adhesion Shear Bond Strength to Enamel or Dentin Test Method

Adhesive shear bond strength to enamel or dentin for a given test sample was evaluated by the following procedure.

Preparation of Teeth. Bovine incisal teeth, free of soft tissue, were embedded in circular acrylic disks. The embedded teeth were stored in water in a refrigerator prior to use. In preparation for adhesive testing, the embedded teeth were ground to expose a flat enamel or dentin surface using 120-grit sandpaper mounted on a lapidary wheel. Further grinding and polishing of the tooth surface was done manually using 320-grit sandpaper. The teeth were continuously rinsed with water during the grinding process. The polished teeth were stored in deionized water and used for testing within 2 hours after polishing. The teeth were allowed to warm in a 36°C oven to between room temperature (23°C) and 36°C before use.

Teeth Treatment. An adhesive test sample was applied with a dental applicator brush over the entire surface of the prepared enamel or dentin surface and rubbed in with moderate finger pressure for 20 seconds. Then a stream of compressed air was blown on until no more moving liquid could be seen. After light curing for 10 seconds with a dental curing light (Elipar™ Trilight, 3M ESPE AG, Seefeld, Germany, light intensity: approx. 800 mW / cm²), the applicator brush was rewetted with the adhesive, and a second layer of adhesive was applied without rubbing in. This layer was thinned for 2 seconds only with a gentle stream of air. Then, the adhesive coating was light cured again for 10 seconds with an Elipar™ Trilight dental curing light. A 2.5 mm thick Teflon mold with a hole approximately 4.7 mm in diameter was clamped to the embedded tooth such that the hole in the mold exposed part of the adhesively prepared tooth surface. A composite material, i.e. an epoxy-based filling material as described in WO 01/10388, page 12, or A3 shade of FILTEK™ Z250 Universal Restorative (3M ESPE Dental Products, St. Paul, MN), was filled into the hole such that the hole was completely filled, but not overfilled, and light cured for 40 seconds in the case of the epoxy based filling material, or 20 seconds in the case of the FILTEK Z250 material to form a "button" that was adhesively attached to the tooth.

Adhesive Bond Strength Testing. The adhesive strength of a cured test example was evaluated by mounting the assembly (described above) in a holder clamped in the jaws of Zwick Universal testing machine (Zwick Z010, Zwick GmbH, Ulm, Germany) with the polished tooth surface oriented parallel to the direction of pull. A loop of orthodontic wire (0.71 mm diameter) was placed around the composite button adjacent to the polished tooth surface. The ends of the orthodontic wire were clamped in the pulling jaw of the Zwick apparatus and pulled at a crosshead speed of 2 mm/min, thereby placing the adhesive bond in shear stress. The force in Newtons (N) at which the bond failed was recorded, and this number was converted to a force per unit area (MPa) using the known surface area of the button. Each reported value of adhesion to enamel or adhesion to dentin represents the average of 5 replicates.

### Rheology measurements

Viscosity vs Shear Rate Curve. The viscosity was measured using a Bohlin CVO 120 rheometer (Bohlin Instruments, NJ) with a plate/plate geometry under controlled shear rate at 23°C. The plate diameter was 40 mm, and the separation between the plates 200 µm. The shear rate was ramped up from 0.5 to 1000 s⁻¹ and in reverse rate down to 0.5 from 1000 s⁻¹. This shear rate ramping took place every 10 seconds, with a 10 second delay time and 10 s integration time, for a total of 20 logarithmically-spaced shear rate steps per run (a run being defined as both increasing and decreasing shear direction).

### Examples 1 - 43

For a dental composition consisting of part A and part B two solutions A and B were prepared. Solution A consists of HEMA (30 parts), water (30 parts), VBP (30.1 parts), DPIPF6 (4.9 parts), Brij 35 (4.9 parts), BHT (0.1 parts). Solutions B1 - 43 were prepared consisting of an acid (b) (amount according to Table 1), BisEMA (amount according to Table 1), CPQ (1.25 parts), EDMAB (2 parts), and BHT (0.125 parts).

Both parts were mixed together at a ratio of 1.000 gram of solutions B1 - 43 to 0.250 grams of solution A. As a test for the handling properties and the viscosity of the compositions the resulting mixtures were checked visually for gel formation.

The formation of a gel by mixing the two parts A and B of the dental composition indicates an increase in viscosity and improved handling properties. In Table 1, "-" denotes no visible gel formation, "+" denotes a visible gel formation, and "++" denotes a strong gel formation. The formulations with the desirable viscosity and rheological properties are among those denoted with a "+".

### Comparative examples 44 - 47

As comparative examples four mixtures with same ratio of solutions B44-47 and solution A were prepared containing the same ingredients for solution A. Solutions B44 - 47 consist of glacial acetic acid as acid (b) (amount according to Table 1), BisEMA (according to Table 1), CPQ (1.25 parts), EDMAB (2 parts), and BHT (0.125 parts).

**Table 1:**

| Example no. | Solution B | acid (b) | acid (b) (parts) | BisEMA (parts) | gel formation |
|---|---|---|---|---|---|
| 1 | B1 | PM2 | 10,000 | 86,625 | - |
| 2 | B2 | PM2 | 20,000 | 76,625 | + |
| 3 | B3 | PM2 | 30,000 | 66,625 | + |
| 4 | B4 | PM2 | 40,000 | 56,625 | + |
| 5 | B5 | PM2 | 50,000 | 46,625 | + |
| 6 | B6 | PM2 | 60,000 | 36,625 | + |
| 7 | B7 | PM2 | 70,000 | 26,625 | + |
| 8 | B8 | PM2 | 80,000 | 16,625 | + |
| 9 | B9 | PM2 | 90,000 | 6,625 | ++ |
| 10 | B10 | PM2 | 96,625 | 0,000 | ++ |
| 11 | B11 | PGDMA | 10,000 | 86,625 | - |
| 12 | B12 | PGDMA | 20,000 | 76,625 | + |
| 13 | B13 | PGDMA | 30,000 | 66,625 | + |
| 14 | B14 | PGDMA | 40,000 | 56,625 | + |
| 15 | B15 | PGDMA | 50,000 | 46,625 | + |
| 16 | B16 | PGDMA | 60,000 | 36,625 | + |
| 17 | B17 | PGDMA | 70,000 | 26,625 | ++ |
| 18 | B18 | PGDMA | 80,000 | 16,625 | ++ |
| 19 | B19 | PGDMA | 90,000 | 6,625 | ++ |
| 20 | B20 | PGDMA | 96,625 | 0,000 | ++ |
| 21 | B21 | DBSA | 20,000 | 76,625 | + |
| 22 | B22 | DBSA | 40,000 | 56,625 | ++ |
| 23 | B23 | DBSA | 60,000 | 36,625 | ++ |
| 24 | B24 | SEMA | 10,000 | 86,625 | - |
| 25 | B25 | SEMA | 20,000 | 76,625 | - |
| 26 | B26 | SEMA | 30,000 | 66,625 | + |
| 27 | B27 | SEMA | 40,000 | 56,625 | + |
| 28 | B28 | SEMA | 50,000 | 46,625 | ++ |
| 29 | B29 | SEMA | 60,000 | 36,625 | ++ |
| 30 | B30 | SEMA | 70,000 | 26,625 | ++ |
| 31 | B31 | SEMA | 80,000 | 16,625 | ++ |
| 32 | B32 | SEMA | 90,000 | 6,625 | ++ |
| 33 | B33 | SEMA | 96,625 | 0,000 | ++ |
| 34 | B34 | MEMA | 10,000 | 86,625 | - |
| 35 | B35 | MEMA | 20,000 | 76,625 | - |
| 36 | B36 | MEMA | 30,000 | 66,625 | - |
| 37 | B37 | MEMA | 40,000 | 56,625 | + |
| 38 | B38 | MEMA | 50,000 | 46,625 | + |
| 39 | B39 | MEMA | 60,000 | 36,625 | + |
| 40 | B40 | MEMA | 70,000 | 26,625 | + |
| 41 | B41 | MEMA | 80,000 | 16,625 | ++ |
| 42 | B42 | MEMA | 90,000 | 6,625 | ++ |
| 43 | B43 | MEMA | 96,625 | 0,000 | ++ |
| 44 comp. | B44 | AA | 20,000 | 76,625 | - |
| 45 comp. | B45 | AA | 40,000 | 56,625 | - |
| 46 comp. | B46 | AA | 60,000 | 36,625 | - |
| 47 comp. | B47 | AA | 80,000 | 16,625 | - |

As can be seen for examples 1 - 43, the addition of an acid derivative with a stronger acidity than the carboxylic acid functional polymer to Part B leads to an increased viscosity and gel formation of the mixtures. Preferably the acid derivative is present in an amount of at least 20 parts. If the content of an acid derivative with a stronger acidity than the carboxylic acid functional polymer in Part B exceeds 80 parts the viscosity of the mixture became too high for optimal handling of the dental composition. For acetic acid which was used for the comparative examples 44 - 47, no such gel formation was seen.

### Example 48 / Comparative example 49

For solution A, solution B3 and solution B45 and the mixture of one part of solution A and four parts of each of solutions B3 and B45, the viscosity versus shear rate was determined as described above. The results are shown in Table 2. While solution A, B3 and B45 exhibited Newtonian behavior, the mixture of A and B3 revealed shear thinning. At low shear rates, the mixture of solution A and B3 showed a higher viscosity than the each of the solutions.

This example demonstrates that upon mixing of the two solutions of lower viscosity according to the present invention, a composition with a higher viscosity is obtained that exhibits shear thinning. This is very beneficial for the use in the dental field, because dosing and mixing of the solutions is facilitated, pooling of the mixture is avoided, and a smooth, homogeneous film is obtained. Inversely, for the comparative example no. 49 with the mixture of solution A and B45 no such shear thinning was found.

**Table 2.**

| | | | | | | |
|---|---|---|---|---|---|---|
| Example no. | shear rate [s⁻¹] | 0.5 | 1 | 10 | 100 | 1000 |
| 48 | viscosity [Pa.s] of A | 0.3 | 0.2 | 0.2 | 0.2 | 0.2 |
| | viscosity [Pa·s] of B3 | 0.9 | 0.9 | 0.9 | 0.9 | 0.9 |
| | viscosity [Pa·s] of mixture A, B3 | 1.4 | 1.2 | 0.7 | 0.4 | 0.4 |
| 49 comp. | viscosity [Pa·s] of A | 0.3 | 0.2 | 0.2 | 0.2 | 0.2 |
| | viscosity [Pa·s] of B45 comp. | 0.2 | 0.2 | 0.2 | 0.2 | 0.2 |
| | viscosity [Pa·s] of mixture A, B45 | 0.2 | 0.2 | 0.2 | 0.2 | 0.2 |

### Example 50

For solution A and solution B26 and for the mixture of one part of solution A and four parts of solution B26, the viscosity versus shear rate was determined as described above. The results are shown in Table 3. While solution A and B26 exhibited Newtonian behavior, the mixture of A and B26 revealed shear thinning while the shear rate was ramped up. At low shear rates, the mixture showed a strong increase in viscosity with respect to solutions A and B 26. For the decreasing shear rate direction, an increase in the viscosity was observed indicating at lease partly reversible shear thinning.

This example also demonstrates that upon mixing of two solutions of lower viscosity according to the present invention, a composition with a higher viscosity is obtained that exhibits shear thinning.

**Table 3:**

| | | | | | | |
|---|---|---|---|---|---|---|
| Example no. | shear rate [s⁻¹] | 0.5 | 1 | 10 | 100 | 1000 |
| 50 | viscosity [Pa·s] of A | 0.3 | 0.2 | 0.2 | 0.2 | 0.2 |
| | viscosity [Pa·s] of B26 | 1.3 | 1.3 | 1.3 | 1.3 | 1.3 |
| | viscosity [Pa·s] of mixture A, B26 | 8.3 | 6.0 | 2.5 | 1.1 | 0.8 |

### Examples 51 - 58:

Adhesive securing of a methacrylate based composite restorative material (FILTEK Z250) on enamel and dentin of bovine teeth was performed as described above for the 1:4 mixtures of solution A and solutions B3, B6, B13, B16, B26, B29, B36, and B39. The results can be found in Table 4.

**Table 4.**

| Example no. | mixture A, Bx | adhesion on enamel [MPa] | adhesion on dentin [MPa] |
|---|---|---|---|
| 51 | A : B3 | 10.7 | 11.1 |
| 52 | A : B6 | 14.7 | 15.4 |
| 53 | A : B13 | 15.1 | 16.0 |
| 54 | A : B16 | 15.2 | 8.2 |
| 55 | A : B26 | 0.1 | 0.5 |
| 56 | A : B29 | 0.1 | 0.0 |
| 57 | A : B36 | 1.7 | 6.7 |
| 58 | A : B39 | 3.3 | 3.8 |

### Example 59

At a ratio of 1 : 4, solution A was mixed with a solution C consisting of PGDMA (50 parts), PM2 (25 parts), TEGDMA (16.725 parts), PUMA (4.900 parts) CPQ (1.250 parts), EDMAB (2.000 parts), BHT (0.125 parts).

For this composition, mixing of solutions A and C was especially easy, although a strong increase in viscosity was obtained for the mixture. Thus, a preferred embodiment of the invention contains additionally at least one urethane functionalized compound.

The viscosity versus shear rate was determined as described above. The results are shown in Table 5. Both solutions A and C exhibited Newtonian behavior, whereas the mixture of A and C exhibited strong shear thinning. For the decreasing shear rate direction, an increase in the viscosity was observed indicating at least partly reversible shear thinning.

**Table 5:**

| | | | | | | |
|---|---|---|---|---|---|---|
| Example no. | shear rate [s⁻¹] | 0.5 | 1 | 10 | 100 | 1000 |
| 59 | viscosity [Pa·s] of A | 0.3 | 0.2 | 0.2 | 0.2 | 0.2 |
| | viscosity [Pa·s] of C | 0.3 | 0.3 | 0.3 | 0.3 | 0.3 |
| | viscosity [Pa·s] of mixture A, C | 6.0 | 4.1 | 0.5 | 0.2 | 0.2 |

### Examples 60 - 61

Adhesive securing of a methacrylate based composite restorative material (example 60; FILTEK Z250) and of an epoxy based filling material (example 61) as described in WO 01/10388, page 12, was performed with the composition from example 59 according to the method described above. The adhesion values for enamel and dentin are given in Table 6. For this mixture, on all substrates, a smooth homogeneous film was easily obtained.

**Table 6:**

| Example no. | | adhesion on enamel [MPa] | adhesion on dentin [MPa] |
|---|---|---|---|
| 60 | Z250 | 13.0 | 11.0 |
| 61 | epoxy based filling material | 14.1 | 12.1 |

### Example 62

A solution D consisting of HEMA (30 parts), water (30 parts), a 1:1 copolymer of acrylic and maleic acid with an average molecular weight Mw = 12000 (30.1 parts), DPIPF6 (4.9 parts), Brij 35 (4.9 parts), BHT (0.1 parts) was mixed at a ratio of 1 : 4 with solution C.

This composition exhibited very little gel formation upon mixing of solutions D and C, although a very strong increase in viscosity was obtained for the mixture with respect to solutions D and C. The viscosity versus shear rate was determined as described above. The results are shown in Table 7. While solutions D and C exhibited Newtonian behavior, the mixture showed strong shear thinning. For the decreasing shear rate direction, an increase in the viscosity was observed indicating at least partly reversible shear thinning.

**Table 7.**

| | | | | | | |
|---|---|---|---|---|---|---|
| Example no. | shear rate [s⁻¹] | 0.5 | 1 | 10 | 100 | 1000 |
| 62 | viscosity [Pa.s] of D | 0.4 | 0.4 | 0.4 | 0.4 | 0.4 |
| | viscosity [Pa·s] of C | 0.3 | 0.3 | 0.3 | 0.3 | 0.3 |
| | viscosity [Pa·s] of mixture D, C | 13 | 3.6 | 0.4 | 0.2 | 0.2 |

### Example 63

Adhesive securing of an epoxy based filling material as described in WO 01/10388, page 12, was performed with the composition from example 62 according to the method described above. The adhesion values for enamel and dentin are given in Table 8. For the mixture of solutions D and C, on all substrates, a smooth homogeneous film was easily obtained.

**Table 8.**

| Example no. | adhesion [MPa] | enamel | dentin |
|---|---|---|---|
| 63 | epoxy based filling material | 6.4 | 5.0 |

### Examples 64 to 68:

As examples 64 to 65 and as comparative examples 66 to 68, mixtures of PGDMA (100 parts) and solution E were prepared. Solution E1-5 consists of BisEMA (amount according to Table 9), HEMA (amount according to Table 9), VBP (20 parts), water (amount according to Table 9), DPIPF6 (0.5 parts), CPQ (0.5 parts), EDMAB (0.5 parts).

The solutions E1-5 were mixed in a ratio of 1 g of solution E1-5 to 1 g of PGDMA. As a test for the handling properties and the viscosity of the compositions the resulting mixtures were checked visually for gel formation. The formation of a gel by mixing the two parts E1-5 and PGDMA of the dental composition indicates an increase in viscosity and improved handling properties. In Table 9, "-" denotes no visible gel formation, "+" denotes a visible gel formation.

As can be seen from the Table 9 for the comparative examples dental compositions containing above 10 % of water do not exhibit a desired viscosity.

**Table 9**

| example no. | Solution E | BisEMA in solution E (parts) | HEMA in solution E (parts) | water in solution E (parts) | viscosity of B+E |
|---|---|---|---|---|---|
| 64 | E1 | 50 | 20 | 10 | + |
| 65 | E2 | 40 | 20 | 20 | + |
| 66 comp. | E3 | 30 | 20 | 30 | - |
| 67 comp. | E4 | 20 | 20 | 40 | - |
| 68 comp. | E5 | 10 | 20 | 50 | - |

## Claims

1. A dental composition comprising:
(a) at least one carboxylic acid functional polymer,
(b) at least one acid derivative with stronger acidity than the carboxylic acid functional polymer (a), substituted with at least one polymerizable ethylenically unsaturated group,
(c) 0.1 to 10 weight % of water,
wherein the carboxylic acid functional polymer (a) and the acid derivative (b) are present at least in an amount effective that the dental composition exhibit shear thinning and a viscosity from 1.0 to 20 Pa·s, when measured with a plate/plate geometry and at a shear rate of 0.5 to 1 s⁻¹ at 23°C.

2. A dental composition according to claim 1 comprising:
(a) 1 to 30 weight % of at least one carboxylic acid functional polymer, substituted with at least one polymerizable ethylenically unsaturated group,
(b) 10 to 80 weight % of at least one acid derivative with stronger acidity than the carboxylic acid functional polymer (a), substituted with at least one polymerizable ethylenically unsaturated group,
(c) 0.1 to 10 weight % of water,
(d) 1 to 80 weight % of at least one unsaturated monomer and/or prepolymer,
(e) 0.1 to 10 weight % of initiators, stabilizers,
wherein the carboxylic acid functional polymer (a) and the acid derivative (b) are present at least in an amount effective that the dental composition exhibit shear thinning and a viscosity from 1.0 to 20 Pa·s, when measured with a plate/plate geometry and at a shear rate of 0.5 to 1 s⁻¹ at 23°C.

3. A dental composition according to any of the preceding claims comprising 0.1 to 6 weight % of water.

4. A dental composition according to any of the preceding claims wherein the viscosity of the composition at a shear rate of 0.5 to 1 s⁻¹ is from 1.0 to 20 Pas and the viscosity at a shear rate of 1000 s⁻¹ is less than 1.0 Pa s and/or the viscosity at a shear rate of 1000 s⁻¹ is less than 30% of the viscosity at a shear rate of 0.5 s⁻¹ at 23°C.

5. A dental composition according to any of the preceding claims wherein the composition does not comprise any particulate viscosity modifiers such as fumed silica, aluminum oxide and titanium dioxide or a glass powder.

6. A dental composition according to any of the preceding claims wherein the acid derivative (b) is selected from the group consisting of ethylenically unsaturated phosphoric acid esters, ethylenically unsaturated phosphoric acid diesters, ethylenically unsaturated sulfonic acid esters, ethylenically unsaturated dicarboxylic acid monoesters, with a pKₐ of less than 4.75.

7. A dental composition according to any of the preceding claims wherein the acid derivative (b) is selected from the group consisting of (meth)acryloyloxyalkyl-phosphate with the alkyl group having one to five carbon atoms, 5-(meth)acryloyloxy-3-oxa-pentyl phosphate, bis((meth)acryloyloxyalkyl) phosphate, bis(5-(meth)acryloyloxy-3-oxa-pentyl) phosphate, glycerol-di(meth)acrylate-phosphate, bis(glycerol-di(meth)acrylate) phosphate, (trimethylolpropane dimethacrylate) phosphate, bis(trimethylolpropane dimethacrylate) phosphate, pentaerythritol trimethacrylate phosphate, mono((meth)acryloyloxyalkyl)maleate, sulfoalkyl (meth)acrylate, 2-acrylamido-2-methyl-propanesulfonic acid.

8. A dental composition according to any of the preceding claims wherein the acid derivative (b) is selected from (i) a mixture of reaction products of 2-hydroxyethyl methacrylate with phosphorus pentoxide, (ii) a mixture of reaction products of glycerol di(methacrylate) with phosphorus pentoxide, (iii) 2-sulfoethyl methacrylate, (iv) mono(methacryloyloxyethyl)maleate.

9. A dental composition according to any of the preceding claims wherein the acid derivative (b) is selected from (i) a mixture of reaction products of 2-hydroxyethyl methacrylate with phosphorus pentoxide, (ii) a mixture of reaction products of glycerol di(methacrylate) with phosphorus pentoxide.

10. A dental composition according to any of the preceding claims wherein the carboxylic acid functional polymer (a) is selected from the group consisting of homopolymers and copolymers of unsaturated mono-, di- or tricarboxylic acids and/or their anhydrides optionally substituted with at least one ethylenically unsaturated group.

11. A dental composition according to any of the preceding claims wherein the carboxylic acid functional polymer (a) is selected from the group consisting of homopolymers of poly(meth)acrylic acid, copolymers of (meth)acrylic and itaconic acid, (meth)acrylic and maleic acid, methyl vinyl ether and maleic anhydride or maleic acid, ethylene and maleic anhydride or maleic acid, and styrene and maleic anhydride or maleic acid, each polymer having at least one acidic group substituted with a polymerizable ethylenically unsaturated group.

12. A dental composition according to any of the preceding claims wherein the composition contains an urethane functionalized compound substituted with at least one polymerizable ethylenically unsaturated group.

13. A dental composition according to claim 10 wherein the urethane functionalized compound is a step growth polymerization product of Tego® Diol BD-1000, 2,4,4-trimethylhexyl-1,6-diisocyanate and 2-hydroxyethylmethacrylate.

14. A dental composition according to any of the preceding claims wherein the polymerizable ethylenically unsaturated group contains an acrylate, methacrylate; acryl amide and/or methacryl amide group.

15. A dental composition, comprising at least two parts A and B wherein part A comprises:
(a) at least one carboxylic acid functional polymer,
(aa) 0 to 10 weight % of water,
and wherein part B comprises:
(b) at least one acid derivative with stronger acidity than the carboxylic acid functional polymer (a), substituted with at least one polymerizable ethylenically unsaturated group,
(bb) less than 0.5 weight % of water,
wherein the carboxylic acid functional polymer (a) and the acid derivative (b) are present at least in an amount effective that the mixture of part A and part B exhibit shear thinning and a viscosity, that is higher than that of each of the separate parts A and B, when measured with a plate/plate geometry and at a shear rate of 0.5 to 1 s⁻¹ at 23°C.

16. A dental composition according to claim 15 wherein the carboxylic acid functional polymer (a) and the acid derivative (b) are present at least in an amount effective that the mixture of part A and part B exhibit shear thinning and a viscosity of at least 2 Pa·s at a shear rate of 0.5 s⁻¹ at 23°C.

17. A dental composition according to claim 15 or 16 wherein the parts A and B are provided each in an amount that the mixture of the parts gives a dental composition with shear thinning and a viscosity of at least 2 Pas at a shear rate of 0.5 s⁻¹ at 23°C.

18. The dental composition according to any of the preceding claims, wherein the acid derivative (b) is present in an amount of 20 - 80 parts by weight of the total composition.

19. The dental composition according to any of the preceding claims, wherein the amount of the acid derivative (b) and the carboxylic acid functional polymer (a) in the total composition is 20 - 90 parts by weight, preferably 30 - 90 parts by weight.

20. The dental composition according to any of the preceding claims, wherein the carboxylic acid functional polymer (a) is present in an amount of 2 - 15 parts by weight of the total composition.

21. The dental composition according to any of the preceding claims having an adhesion to enamel and/or dentin in the range of 1 - 40 MPa.

22. The dental composition according to any of the preceding claims for use as a dental adhesive, pit and fissure sealant, desensitizer and/or restorative.

23. The dental composition according to any of the preceding claims for use as an adhesive for dental filling materials and/or orthodontic appliances.

24. The dental composition according to any of the preceding claims for use as an adhesive for cationically polymerizable filling materials.

25. Method for preparing a dental adhesive by mixing the components or parts of the dental composition according to any of the preceding claims.

## Patentansprüche

1. Dentalmasse, umfassend:
(a) mindestens ein carbonsäurefunktionelles Polymer,
(b) mindestens ein Säurederivat, das eine stärkere Acidität als das carbonsäurefunktionelle Polymer (a) aufweist und mit mindestens einer polymerisierbaren ethylenisch ungesättigten Gruppe substituiert ist,
(c) 0,1 bis 10 Gew.-% Wasser,
wobei das carbonsäurefunktionelle Polymer (a) und das Säurederivat (b) mindestens in einer derartigen Menge vorliegen, daß die Dentalmasse Scherverdünnung und eine viskosität von 1,0 bis 20 Pa.s bei Messung mit einer Platte-Platte-Geometrie und bei einer Scherrate von 0,5 bis 1 s⁻¹ bei 23°C aufweist.

2. Dentalmasse nach Anspruch 1, umfassend:
(a) 1 bis 30 Gew.-% mindestens eines carbonsäurefunktionellen Polymers, das mit mindestens einer polymerisierbaren ethylenisch ungesättigten Gruppe substituiert ist,
(b) 10 bis 80 Gew.-% mindestens eines Säurederivats, das eine stärkere Acidität als das carbonsäurefunktionelle Polymer (a) aufweist und mit mindestens einer polymerisierbaren ethylenisch ungesättigten Gruppe substituiert ist,
(c) 0,1 bis 10 Gew.-% Wasser,
(d) 1 bis 80 Gew.-% mindestens eines ungesättigten Monomers und/oder Prepolymers,
(e) 0,1 bis 10 Gew.-% Initiatoren, Stabilisatoren,
wobei das carbonsäurefunktionelle Polymer (a) und das Säurederivat (b) mindestens in einer derartigen Menge vorliegen, daß die Dentalmasse Scherverdünnung und eine viskosität von 1,0 bis 20 Pa·s bei Messung mit einer Platte-Platte-Geometrie und bei einer Scherrate von 0,5 bis 1 s⁻¹ bei 23°C aufweist.

3. Dentalmasse nach einem der vorhergehenden Ansprüche, umfassend 0,1 bis 6 Gew.-% Wasser.

4. Dentalmasse nach einem der vorhergehenden Ansprüche, wobei die Viskosität der Masse bei einer Scherrate von 0,5 bis 1 s⁻¹ 1,0 bis 20 Pas beträgt und die Viskosität bei einer Scherrate von 1000 s⁻¹ weniger als 1,0 Pas beträgt und/oder die Viskosität bei einer Scherrate von 1000 s⁻¹ weniger als 30% der viskosität bei einer Scherrate von 0,5 s⁻¹ bei 23°C beträgt.

5. Dentalmasse nach einem der vorhergehenden Ansprüche, wobei die Masse keine teilchenförmigen viskositätsmodifikatoren wie pyrogenes Siliciumdioxid, Aluminiumoxid und Titandioxid oder ein Glaspulver umfaßt.

6. Dentalmasse nach einem der vorhergehenden Ansprüche, wobei das Säurederivat (b) aus der Gruppe bestehend aus ethylenisch ungesättigten Phosphorsäureestern, ethylenisch ungesättigten Phosphorsäurediestern, ethylenisch ungesättigten Sulfonsäureestern und ethylenisch ungesättigten Dicarbonsäuremonoestern mit einem pKₐ-Wert von weniger als 4,75 ausgewählt ist.

7. Dentalmasse nach einem der vorhergehenden Ansprüche, wobei das Säurederivat (b) aus der Gruppe bestehend aus (Meth)acryloyloxyalkyl-phosphat mit einem bis fünf Kohlenstoffatomen in der Alkylgruppe, 5-(Meth)acryloyloxy-3-oxapentyl-phosphat, Bis((meth)acryloyloxyalkyl)phosphat, Bis(5-(meth)acryloyloxy-3-oxapentyl)phosphat, Glycerindi(meth)acrylatphosphat, Bis(glycerin-di(meth)acrylat)phosphat, (Trimethylolpropan-dimethacrylat)phosphat, Bis(trimethylolpropan-dimethacrylat)phosphat, Pentaerythrittrimethacrylatphosphat, Mono((meth)acryloyloxy-alkyl)maleat, Sulfoalkyl(meth)acrylat und 2-Acryl-amido-2-methylpropansulfonsäure ausgewählt ist.

8. Dentalmasse nach einem der vorhergehenden Ansprüche, wobei das Säurederivat (b) unter (i) einem Gemisch der Reaktionsprodukte von 2-Hydroxyethylmethacrylat mit Phosphorpentoxid, (ii) einem Gemisch der Reaktionsprodukte von Glycerin-di(meth)acrylat mit Phosphorpentoxid, (iii) 2-Sulfoethylmethacrylat und (iv) Mono(meth)acryloyl-oxyethyl)maleat ausgewählt ist.

9. Dentalmasse nach einem der vorhergehenden Ansprüche, wobei das Säurederivat (b) unter (i) einem Gemisch der Reaktionsprodukte von 2-Hydroxyethylmethacrylat mit Phosphorpentoxid und (ii) einem Gemisch der Reaktionsprodukte von Glycerindi(meth)acrylat mit Phosphorpentoxid ausgewählt ist.

10. Dentalmasse nach einem der vorhergehenden Ansprüche, wobei das carbonsäurefunktionelle Polymer (a) aus der Gruppe bestehend aus Homopolymeren und Copolymeren von ungesättigten Mono-, Di- oder Tricarbonsäuren und/oder ihren Anhydriden, die gegebenenfalls mit mindestens einer ethylenisch ungesättigten Gruppe substituiert sind, ausgewählt ist.

11. Dentalmasse nach einem der vorhergehenden Ansprüche, wobei das carbonsäurefunktionelle Polymer (a) aus der Gruppe bestehend aus Homopolymeren von Poly(meth)acrylsäure, Copolymeren von (Meth)acrylsäure und Itaconsäure, (Meth)acrylsäure und Maleinsäure, Methylvinylether und Maleinsäureanhydrid oder Maleinsäure, Ethylen und Maleinsäureanhydrid oder Maleinsäure und Styrol und Maleinsäureanhydrid oder Maleinsäure ausgewählt ist, wobei jedes Polymer mindestens eine mit einer polymerisierbaren ethylenisch ungesättigten Gruppe substituierte saure Gruppe aufweist.

12. Dentalmasse nach einem der vorhergehenden Ansprüche, wobei die Masse eine urethanfunktionalisierte Verbindung, die mit mindestens einer polymerisierbaren ethylenisch ungesättigten Gruppe substituiert ist, enthält.

13. Dentalzusammensetzung nach Anspruch 10, wobei es sich bei der urethanfunktionalisierten Verbindung um ein Stufenwachstumspolymerisationsprodukt von Tego® Diol BD-1000, 2,4,4-Trimethylhexyl-1,6-diisocyanat und 2-Hydroxethylmethacrylat handelt.

14. Dentalmasse nach einem der vorhergehenden Ansprüche, wobei die polymerisierbare ethylenisch ungesättigte Gruppe eine Acrylat-, Methacrylat-, Acrylamid- und/oder Methacrylamidgruppe enthält.

15. Dentalmasse, umfassend mindestens zwei Teile A und B, wobei Teil A:
(a) mindestens ein carbonsäurefunktionelles Polymer,
(aa) 0 bis 10 Gew.-% Wasser
umfaßt und Teil B:
(b) mindestens ein Säurederivat, das eine stärkere Acidität als das carbonsäurefunktionelle Polymer (a) aufweist und mit mindestens einer polymerisierbaren ethylenisch ungesättigten Gruppe substituiert ist,
(bb) weniger als 0,5 Gew.-% Wasser
umfaßt,
wobei das carbonsäurefunktionelle Polymer (a) und das Säurederivat (b) mindestens in einer derartigen Menge vorliegen, daß die Mischung von Teil A und Teil B Scherverdünnung und eine höhere Viskosität als jeder der separaten Teile A und B bei Messung mit einer Platte-Platte-Geometrie und bei einer Scherrate von 0,5 bis 1 s⁻¹ bei 23°C aufweist.

16. Dentalmasse nach Anspruch 15, wobei das carbonsäurefunktionelle Polymer (a) und das Säurederivat (b) mindestens in einer derartigen Menge vorliegen, daß die Mischung von Teil A und Teil B Scherverdünnung und eine Viskosität von mindestens 2 Pa·s bei einer Scherrate von 0,5 s⁻¹ bei 23°C aufweist.

17. Dentalmasse nach Anspruch 15 oder 16, wobei die Teile A und B jeweils in einer derartigen Menge bereitgestellt werden, daß die Mischung der Teile eine Dentalmasse mit Scherverdünnung und einer Viskosität von mindestens 2 Pas bei einer Scherrate von 0,5 s⁻¹ bei 23°C liefert.

18. Dentalmasse nach einem der vorhergehenden Ansprüche, wobei das Säurederivat (b) in einer Menge von 20 - 80 Gewichtsteilen, bezogen auf die gesamte Zusammensetzung, vorliegt.

19. Dentalmasse nach einem der vorhergehenden Ansprüche, wobei die Menge des Säurederivats (b) und des carbonsäurefunktionellen Polymers (a) in der gesamten Zusammensetzung 20 - 90 Gewichtsteile und vorzugsweise 30 - 90 Gewichtsteile beträgt.

20. Dentalmasse nach einem der vorhergehenden Ansprüche, wobei das carbonsäurefunktionelle Polymer (a) in einer Menge von 2 - 15 Gewichtsteilen, bezogen auf die gesamte Zusammensetzung, vorliegt.

21. Dentalmasse nach einem der vorhergehenden Ansprüche mit einer Zahnschmelz- und/oder Dentinhaftung im Bereich von 1 - 40 MPa.

22. Dentalmasse nach einem der vorhergehenden Ansprüche zur Verwendung als Dentalklebstoff, Loch- und Fissurenversiegler, Desensibilisator und/oder Restaurationsmaterial.

23. Dentalmasse nach einem der vorhergehenden Ansprüche zur Verwendung als Klebstoff für Zahnfüllungsmaterialien und/oder kieferorthopädische Geräte.

24. Dentalmasse nach einem der vorhergehenden Ansprüche zur Verwendung als Klebstoff für kationisch polymerisierbare Füllungsmaterialien.

25. Verfahren zur Herstellung einer Dentalmasse durch Mischen der Komponenten oder Teile der Dentalmasse nach einem der vorhergehenden Ansprüche.

## Revendications

1. Composition dentaire comprenant :
(a) au moins un polymère à fonctionnalité d'acide carboxylique,
(b) au moins un dérivé d'acide présentant une plus forte acidité que le polymère à fonctionnalité d'acide carboxylique (a), substitué par au moins un groupe polymérisable à insaturation éthylénique,
(c) de 0,1 à 10 % en poids d'eau,
dans laquelle le polymère à fonctionnalité d'acide carboxylique (a) et le dérivé d'acide (b) sont présents au moins dans une quantité efficace pour que la composition dentaire soit capable d'être fluidifiée par cisaillement et présente une viscosité de 1,0 à 20 Pa·s, mesurée avec une géométrie plaque/plaque et à une vitesse de cisaillement de 0,5 à 1 s⁻¹ à 23 ºC.

2. Composition dentaire selon la revendication 1, comprenant :
(a) de 1 à 30 % en poids d'au moins un polymère à fonctionnalité d'acide carboxylique, substitué par au moins un groupe polymérisable à insaturation éthylénique,
(b) de 10 à 80 % en poids d'au moins un dérivé d'acide présentant une plus forte acidité que le polymère à fonctionnalité d'acide carboxylique (a), substitué par au moins un groupe polymérisable à insaturation éthylénique,
(c) de 0,1 à 10 % en poids d'eau,
(d) de 1 à 80 % en poids d'au moins un monomère et/ou prépolymère insaturé,
(e) de 0,1 à 10 % en poids d'initiateurs, de stabilisants,
dans laquelle le polymère à fonctionnalité d'acide carboxylique (a) et le dérivé d'acide (b) sont présents au moins dans une quantité efficace pour que la composition dentaire soit capable d'être fluidifiée par cisaillement et présente une viscosité de 1,0 à 20 Pa·s, mesurée avec une géométrie plaque/plaque et à une vitesse de cisaillement de 0,5 à 1 s⁻¹ à 23 ºC.

3. Composition dentaire selon l'une quelconque des revendications précédentes, comprenant de 0,1 à 6 % en poids d'eau.

4. Composition dentaire selon l'une quelconque des revendications précédentes, dans laquelle la viscosité de la composition à une vitesse de cisaillement de 0,5 à 1 s⁻¹ est de 1,0 à 20 Pa.s, et la viscosité à une vitesse de cisaillement de 1 000 s⁻¹ est inférieure à 1,0 Pa·s, et/ou la viscosité à une vitesse de cisaillement de 1 000 s⁻¹ est inférieure à 30 % de la viscosité à une vitesse de cisaillement de 0,5 s⁻¹ à 23 ºC.

5. Composition dentaire selon l'une quelconque des revendications précédentes, la composition ne comprenant aucun modificateur de viscosité particulaire, par exemple la silice pyrogénée, l'oxyde d'aluminium et le dioxyde de titane ou une poudre de verre.

6. Composition dentaire selon l'une quelconque des revendications précédentes, dans laquelle le dérivé d'acide (b) est sélectionné dans le groupe constitué d'esters d'acide phosphorique à insaturation éthylénique, de diesters d'acide phosphorique à insaturation éthylénique, d'esters d'acide sulfonique à insaturation éthylénique, de monoesters d'acide dicarboxylique à insaturation éthylénique, ayant une valeur pKₐ inférieure à 4,75.

7. Composition dentaire selon l'une quelconque des revendications précédentes, dans laquelle le dérivé d'acide (b) est sélectionné dans le groupe constitué de phosphate de (méth)acryloyloxyalkyle dont le groupe alkyle contient de 1 à 5 atomes de carbone, de phosphate de 5-(méth)acryloyloxy-3-oxa-pentyle, de phosphate de bis((méth)acryloyloxyalkyle), de phosphate de bis(5-(méth)acryloyloxy-3-oxa-pentyle), de phosphate de glycérol-di(méth)acrylate, de phosphate de bis(glycérol-di-(méth)acrylate), de phosphate de (diméthacrylate de triméthylolpropane), de phosphate de bis(diméthacrylate de triméthylolpropane), de phosphate de triméthacrylate de pentaérythritol, de maléate de mono((méth)acryloyloxyalkyle), de (méth)acrylate de sulfoalkyle, d'acide 2-acrylamido-2-méthyl-propanesulfonique.

8. Composition dentaire selon l'une quelconque des revendications précédentes, dans laquelle le dérivé d'acide (b) est sélectionné parmi (i) un mélange de produits réactionnels de méthacrylate de 2-hydroxyéthyle avec du pentoxyde de phosphore, (ii) un mélange de produits réactionnels de di(méthacrylate) de glycérol avec du pentoxyde de phosphore, (iii) le méthacrylate de 2-sulfoéthyle, (iv) le maléate de mono(méthacryloyloxyéthyle).

9. Composition dentaire selon l'une quelconque des revendications précédentes, dans laquelle le dérivé d'acide (b) est sélectionné parmi (i) un mélange de produits réactionnels de méthacrylate de 2-hydroxyéthyle avec du pentoxyde de phosphore, (ii) un mélange de produits réactionnels de di(méthacrylate) de glycérol avec du pentoxyde de phosphore.

10. Composition dentaire selon l'une quelconque des revendications précédentes, dans laquelle le polymère à fonctionnalité d'acide carboxylique (a) est sélectionné dans le groupe constitué d'homopolymères et de copolymères d'acides monocarboxyliques, dicarboxyliques ou tricarboxyliques insaturés et/ou leurs anhydrides éventuellement substitués par au moins un groupe à insaturation éthylénique.

11. Composition dentaire selon l'une quelconque des revendications précédentes, dans laquelle le polymère à fonctionnalité d'acide carboxylique (a) est sélectionné dans le groupe constitué d'homopolymères d'acide poly(méth)acrylique, de copolymères d'acide (méth)acrylique et itaconique, d'acide (méth)acrylique et maléique, d'éther méthylvinylique et d'anhydride maléique ou d'acide maléique, d'éthylène et d'anhydride maléique ou d'acide maléique, et de styrène et d'anhydride maléique ou d'acide maléique, chaque polymère comportant au moins un groupe acide substitué par un groupe polymérisable à insaturation éthylénique.

12. Composition dentaire selon l'une quelconque des revendications précédentes, la composition contenant un composé à fonctionnalité uréthane substitué par au moins un groupe polymérisable à insaturation éthylénique.

13. Composition dentaire selon la revendication 10, dans laquelle le composé à fonctionnalité uréthane est un produit de polymérisation à croissance par étapes de Tego® Diol BD-1000, de 2,4,4-triméthylhexyl-1,6-diisocyanate et de 2-hydroxyéthylméthacrylate.

14. Composition dentaire selon l'une quelconque des revendications précédentes, dans laquelle le groupe polymérisable à insaturation éthylénique contient un groupe acrylate, méthacrylate, acrylamide et/ou méthacrylamide.

15. Composition dentaire, comprenant au moins deux parties A et B, dans laquelle la partie A comprend :
(a) au moins un polymère à fonctionnalité d'acide carboxylique,
(aa) de 0 à 10 % en poids d'eau,
et dans laquelle la partie B comprend :
(b) au moins un dérivé d'acide présentant une plus forte acidité que le polymère à fonctionnalité d'acide carboxylique (a), substitué par au moins un groupe polymérisable à insaturation éthylénique,
(bb) moins de 0,5 % en poids d'eau,
dans laquelle le polymère à fonctionnalité d'acide carboxylique (a) et le dérivé d'acide (b) sont présents au moins dans une quantité efficace pour que le mélange de la partie A et de la partie B soit capable d'être fluidifié par cisaillement et présente une viscosité qui est supérieure à celle de chacune des parties A et B séparées, mesurée avec une géométrie plaque/plaque et à une vitesse de cisaillement de 0,5 à 1 s⁻¹ à 23 ºC.

16. Composition dentaire selon la revendication 15, dans laquelle le polymère à fonctionnalité d'acide carboxylique (a) et le dérivé d'acide (b) sont présents au moins dans une quantité efficace pour que le mélange de la partie A et de la partie B soit capable d'être fluidifié par cisaillement et présente une viscosité d'au moins 2 Pa·s à une vitesse de cisaillement de 0,5 s⁻¹ à 23 ºC.

17. Composition dentaire selon la revendication 15 ou 16, dans laquelle les parties A et B sont fournies chacune dans une quantité telle que le mélange des parties produise une composition dentaire capable d'être fluidifiée par cisaillement et présentant une viscosité d'au moins 2 Pa·s à une vitesse de cisaillement de 0,5 s⁻¹ à 23 ºC.

18. Composition dentaire selon l'une quelconque des revendications précédentes, dans laquelle le dérivé d'acide (b) est présent dans une quantité de 20 - 80 parties pondérales de la composition totale.

19. Composition dentaire selon l'une quelconque des revendications précédentes, dans laquelle la quantité du dérivé d'acide (b) et du polymère à fonctionnalité d'acide carboxylique (a) dans la composition totale est de 20 - 90 parties pondérales, préférablement de 30 - 90 parties pondérales.

20. Composition dentaire selon l'une quelconque des revendications précédentes, dans laquelle le polymère à fonctionnalité d'acide carboxylique (a) est présent dans une quantité de 2 - 15 parties pondérales de la composition totale.

21. Composition dentaire selon l'une quelconque des revendications précédentes, ayant une adhérence à l'émail et/ou à la dentine de 1 - 40 MPa.

22. Composition dentaire selon l'une quelconque des revendications précédentes, destinée à une utilisation comme adhésif dentaire, comme agent d'étanchéification pour les puits et les fissures, comme désensibilisateur et/ou comme matériau de restauration.

23. Composition dentaire selon l'une quelconque des revendications précédentes, destinée à une utilisation comme adhésif pour des matériaux d'obturation dentaire et/ou des appareils orthodontiques.

24. Composition dentaire selon l'une quelconque des revendications précédentes, destinée à une utilisation comme adhésif pour des matériaux d'obturation polymérisables par polymérisation cationique.

25. Procédé de préparation d'un adhésif dentaire par mélange des composants ou des parties de la composition dentaire selon l'une quelconque des revendications précédentes.
